(19) Europäisches Patentamt
European Patent Office
Office européen des brevets

(11) **EP 1 203 226 B1**

(12) **FASCICULE DE BREVET EUROPEEN**

(45) Date de publication et mention
de la délivrance du brevet:
**17.01.2007 Bulletin 2007/03**

(51) Int Cl.:
**G01N 27/12** *(2006.01)*

(21) Numéro de dépôt: **00949040.0**

(86) Numéro de dépôt international:
**PCT/CH2000/000424**

(22) Date de dépôt: **09.08.2000**

(87) Numéro de publication internationale:
**WO 2001/013101 (22.02.2001 Gazette 2001/08)**

(54) **DETECTEUR DE GAZ**

GASSENSOR

GAS SENSOR

(84) Etats contractants désignés:
**CH DE FR GB LI**

(30) Priorité: **13.08.1999 FR 9910470**

(43) Date de publication de la demande:
**08.05.2002 Bulletin 2002/19**

(73) Titulaire: **Microchemical Systems S.A.**
**2035 Corcelles (CH)**

(72) Inventeurs:
• **LESCOUZERES, Lionel**
**F-31100 Toulouse (FR)**
• **SAUVAN, Muriel**
**CH-2013 Colombier (CH)**
• **BERTHOUD, Christophe**
**CH-1206 Genève (CH)**

(74) Mandataire: **GLN**
**Rue du Puits-Godet 8a**
**2000 Neuchâtel (CH)**

(56) Documents cités:
**US-A- 3 906 473          US-A- 4 567 475**
**US-A- 4 896 143**

## Description

La présente invention concerne un détecteur de gaz et, plus particulièrement, un détecteur du type comportant:

- un capteur comprenant une couche sensible d'oxyde métallique destinée à être mise en contact avec un mélange gazeux contenant le gaz recherché,
- des moyens de chauffage pour faire varier cycliquement la température de la couche sensible entre une température inférieure et une température supérieure,
- des moyens de mesure pour obtenir plusieurs valeurs de la résistance de la couche pendant le cycle de chauffage, et
- une électronique de détermination de la concentration du gaz dans le mélange à partir de ces valeurs.

Les détecteurs de ce type sont bien connus de l'homme de l'art. Ils permettent notamment de déterminer la concentration de monoxyde de carbone (CO) dans l'atmosphère ambiante. Par exemple, selon le brevet US 3,906,473, un capteur à l'oxyde d'étain est chauffé rapidement à une température supérieure durant un bref instant puis refroidi jusqu'à une température d'équilibre inférieure, la mesure de la concentration étant dérivée de la valeur de la résistance de la couche d'oxyde à la température d'équilibre inférieure. Ce procédé est sensible notamment à la variation de la température ambiante et à l'humidité de l'air. C'est pourquoi, il est nécessaire d'introduire des compensations qui affectent la précision et augmentent le coût du dispositif.

Pour pallier cet inconvénient, le brevet US 4,896,143 enseigne d'effectuer une mesure de la résistance à deux températures d'équilibre, l'une supérieure, l'autre inférieure. La concentration en CO est ensuite déterminée à l'aide d'un microprocesseur selon une formule qui, de manière empirique, combine les valeurs de la résistance de la couche sensible aux deux températures. En procédant ainsi, il est possible de déterminer cette concentration avec une humidité relative variant de 15 à 90% et une température comprise entre 0 et 50°C.

Le brevet US 4,567,475 décrit une réalisation du même type, dans laquelle la résistance de la couche sensible est mesurée en permanence.

Dans la pratique, il est apparu que d'autres gaz pouvaient modifier considérablement les paramètres mesurés. Ainsi, la présence d'hydrocarbures ou de monoxyde d'azote dans l'atmosphère ambiante change la valeur des résistances, ce qui affecte la précision de mesure. C'est pourquoi, il a été proposé d'utiliser plusieurs capteurs présentant des sensibilités différentes aux gaz présents. Ces capteurs sont reliés à un système informatique qui analyse les signaux reçus de chacun et, par comparaison des résultats enregistrés, détermine les concentrations des différents gaz. Un tel système est cependant onéreux du fait qu'il nécessite plusieurs capteurs. Le but principal de la présente invention est de réaliser un détecteur permettant de déterminer de manière précise et sûre la concentration d'un ou de plusieurs gaz dans un mélange gazeux, avec un minimum de moyens et à faible coût.

Pour atteindre ce but, le détecteur selon l'invention, comme défini dans la revendication 1, est caractérisé en ce que les moyens de mesure sont agencés de manière à recueillir, lors de chaque cycle, uniquement:

- des valeurs de la résistance de la couche sensible à deux températures intermédiaires aux températures d'équilibre, à des instants prédéterminés une fois lorsque la température est croissante et une fois lorsqu'elle est décroissante, et
- des valeurs de la résistance de la couche sensible aux deux températures d'équilibre.

L'électronique comprend des moyens de traitement pour fournir, à partir des valeurs de résistance recueillies, des informations représentatives de la forme de la courbe de variation de la résistance au cours d'un cycle et des moyens d'analyse de ces informations pour en déduire la concentration du gaz recherché dans le mélange.

Plus précisément, ces informations comprennent, d'une part, la différence X entre les résistances mesurées à la température d'équilibre supérieure et à la température croissante et, d'autre part, la différence Y entre les résistances mesurées à la température d'équilibre inférieure et à la température décroissante. En outre, l'électronique comprend une mémoire destinée à contenir des données de référence représentatives de la forme de la courbe de variation de la résistance pour différentes concentrations du gaz à détecter. Les moyens d'analyse sont agencés pour comparer les informations fournies par les moyens de traitement aux données de référence pour en déduire la concentration de gaz dans le mélange.

De manière avantageuse, la mémoire contient des paramètres représentatifs d'une pluralité de droites découpant en plusieurs domaines un diagramme en coordonnées cartésiennes avec, en abscisse, la valeur de X et, en ordonnée, la valeur de Y, obtenues avec des mélanges gazeux contenant le gaz recherché à plusieurs concentrations connues. Les paramètres contenus dans la mémoire sont alors l'ordonnée à l'origine et la pente de chacune des droites.

De préférence, les moyens d'analyse sont agencés de manière à identifier le domaine du diagramme dans lequel se situe le point de coordonnées X et Y correspondant aux résistances mesurées.

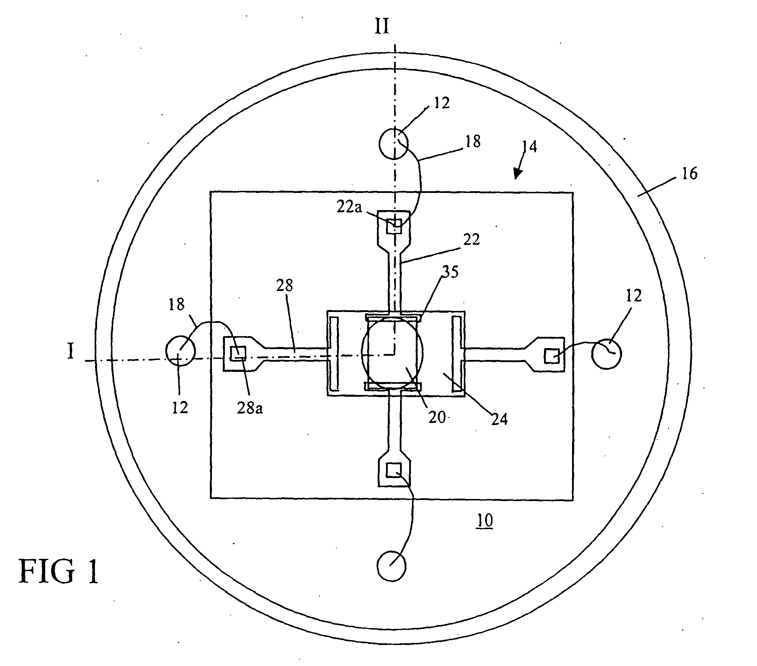

L'expérience a montré que les résultats étaient particulièrement favorables lorsque l'inertie thermique du capteur était faible, de manière que la couche sensible atteigne sa température d'équilibre en un temps inférieur à la seconde. De telles conditions sont obtenues lorsque le capteur est formé d'un empilement de couches comprenant au moins un substrat, un corps de chauffe occupant une partie centrale du substrat, et une couche sensible d'oxyde métallique disposée au moins partiellement au-dessus du corps de coupe selon la ligne brisée I - II de la figure 1;

- la figure 3 est un schéma de principe du détecteur selon l'invention;
- les figures 4 et 5 montrent les courbes de variation de la résistance de la couche sensible du capteur en fonction du temps pour différentes concentrations de l'atmosphère respectivement en monoxyde de carbone (CO) et en méthane (CH4), et
- les figures 6 et 7 sont respectivement un diagramme et un arbre logique illustrant la manière de déterminer les caractéristiques d'un mélange gazeux analysé.

Le capteur de la figure 1, qui équipe un détecteur de gaz selon l'invention, comporte une embase 10, quatre poteaux 12 traversant l'embase et destinés à former les bornes électriques du capteur, et une puce 14 fixée sur l'embase 10 et recouverte d'un boîtier de protection 16 (représenté partiellement). La puce est reliée aux poteaux 12 par des fils conducteurs 18 soudés à chacune de leurs extrémités.

La puce 14 comprend, sur sa face supérieure, une couche sensible 20 formée d'un oxyde métallique, par exemple du dioxyde d'étain ($SnO_2$). Cette couche est en contact, par une partie de sa périphérie, avec deux pistes conductrices 22 destinées à injecter le courant électrique de mesure. Leurs extrémités opposées à la zone de contact avec la couche 20 comportent chacune une plage 22a sur laquelle est soudé le fil 18 destiné à assurer la liaison avec un poteau 12.

Un corps de chauffe 24, séparé de la couche sensible 20 par une couche isolante 26, permet de chauffer le capteur par effet Joule à la température désirée. Il est alimenté électriquement par des pistes conductrices 28 en contact avec ses extrémités. Les pistes 28 comportent chacune, à leur extrémité opposée au corps de chauffe 24, une plage 28a sur laquelle est soudé un fil 18 destiné à assurer la liaison avec un poteau 12.

Le tout est disposé sur un substrat 30 revêtu d'une membrane 32. En outre, une couche de passivation 34 est disposée sur la face supérieure pour recouvrir la puce 14 à l'exception des plages 22a et 28a et d'une découpe 36 essentiellement occupée par la couche sensible 20. Afin de réduire l'inertie thermique de l'ensemble, le substrat 30 est creusé sous le corps de chauffe, jusqu'à la membrane 32, pour former un dégagement 38.

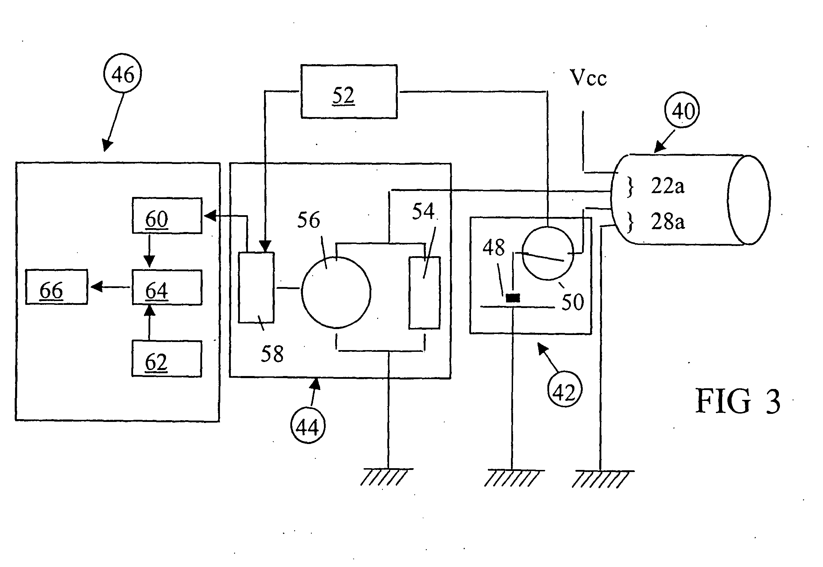

On se référera maintenant à la figure 3 qui représente une forme de réalisation d'un détecteur de gaz selon l'invention. Il comporte essentiellement:

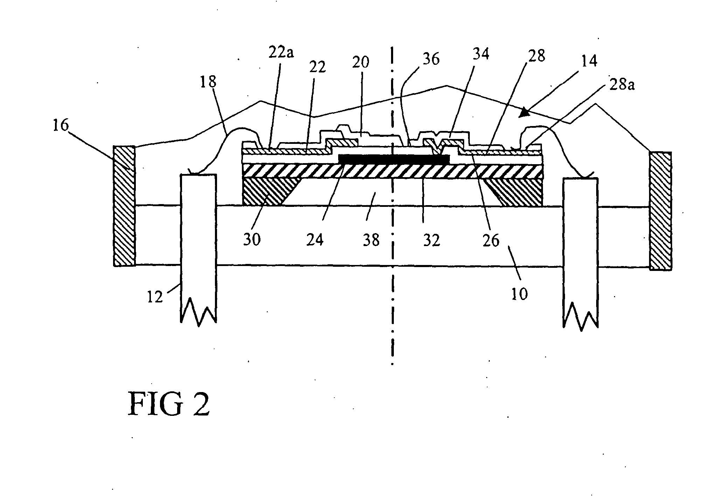

- un capteur à oxyde métallique 40, tel que précédemment décrit en regard des figures 1 et 2;
- des moyens de chauffage 42 pour faire varier cycliquement, à l'aide du corps de chauffe 24, la température de la couche sensible d'oxyde métallique 20 du capteur 40 entre un niveau d'équilibre inférieur et un niveau d'équilibre supérieur;
- des moyens de mesure 44 pour obtenir plusieurs valeurs de la résistance de la couche sensible 20 pendant le cycle de chauffage; et
- une électronique 46 de détermination de la concentration d'un gaz recherché à partir de ces valeurs de résistance.

Les deux poteaux 12 reliés aux plages 22a sont connectés respectivement à une source de tension Vcc et aux moyens de mesure 44. Les deux poteaux 12 reliés aux plages 28a sont connectés respectivement aux moyens de chauffage 42 et à la masse.

Les moyens de chauffage 42 comprennent, montés en série entre une plage 28a du capteur 40 et la masse, une source de courant 48 et un interrupteur 50 piloté par une base de temps 52 de manière à alimenter le corps de chauffe 24 pour faire varier cycliquement la température de la couche sensible 20.

A titre indicatif, lorsque le corps de chauffe 24 est alimenté, le niveau d'équilibre supérieur de la température est d'environ 450°C.

En variante, afin de mieux maîtriser le niveau d'équilibre inférieur de la température, celui-ci correspond, non pas à un arrêt pur et simple du chauffage, mais à un chauffage réduit. Typiquement, ce niveau d'équilibre inférieur est d'environ 50°C.

La période des cycles et la durée de chauffage dépendent du type de gaz recherché. Typiquement, lorsqu'il s'agit de monoxyde d'azote, la période est de 15 secondes et la durée de chauffage de 5 secondes. Dans le cas du méthane, ces valeurs sont respectivement de 10 et 3 secondes.

Les moyens de mesure 44 comprennent, montés en parallèle entre une plage 22a du capteur et la masse, une résistance de référence 54 et un ohmmètre 56 délivrant un signal représentatif de la résistance de la couche sensible du capteur 40. Ce signal sort des moyens de mesure 44 à travers une porte 58 qui, sous les ordres de la base de temps 52, permet de disposer, à chaque cycle, de plusieurs valeurs de la résistance à des instants prédéterminés.

L'électronique 46 de détermination de la concentration comporte des moyens de traitement 60 connectés pour recevoir le signal délivré par les moyens de mesure 44 et tirant de celui-ci, pour chaque cycle, des informations représentatives de la forme de la courbe de variation de la résistance de la couche sensible. La nature de ces informations sera décrite plus bas.

L'électronique 46 comporte aussi:

- une mémoire 62 contenant des données de référence représentatives de la forme de la courbe de variation de la résistance de la couche sensible pour différentes concentrations du gaz recherché;

- des moyens d'analyse 64 pour comparer les informations fournies par les moyens de traitement 60 et les données de référence contenues dans la mémoire 62, puis en déduire la concentration du gaz; et enfin
- un dispositif d'affichage 66 de cette concentration.

L'électronique 16 est avantageusement constituée d'un microprocesseur. La description qui vient d'en être faite concerne donc les différentes fonctions exécutées.

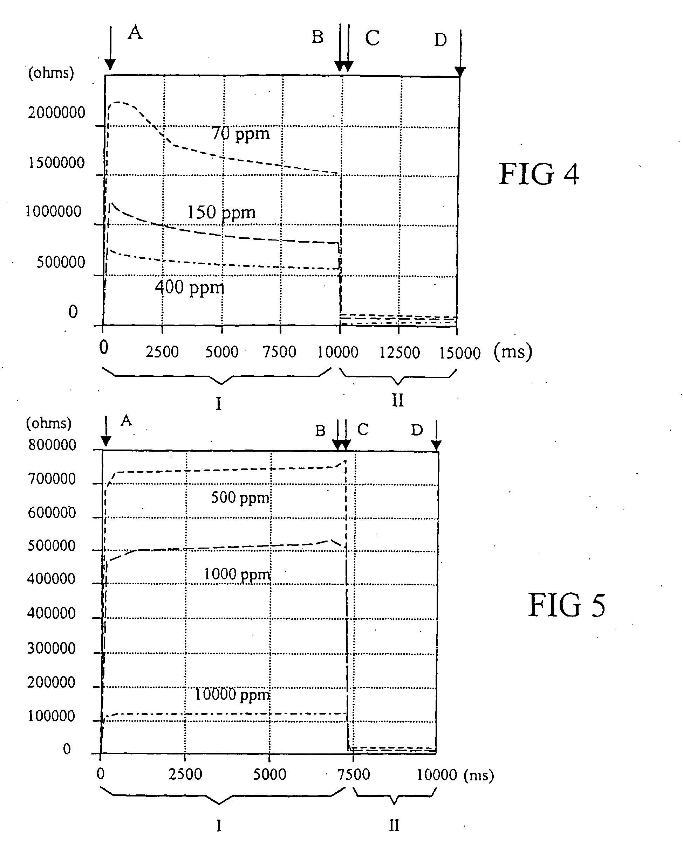

Bien entendu, il est particulièrement avantageux que la forme de la courbe de variation de la résistance soit déterminée à partir d'un minimum de valeurs de celle-ci. On se référera maintenant aux figures 4 et 5 pour expliquer la façon dont sont choisis, au cours d'un cycle, les instants auxquels les mesures sont effectuées.

Ces figures montrent les courbes de variation de la résistance de la couche sensible 20 du capteur en fonction du temps sur un cycle pour différentes concentrations de l'atmosphère en monoxyde de carbone (figure 4) et en méthane (figure 5). Au début de la phase I, qui dure respectivement 10 et 7 secondes, le chauffage est arrêté ou réduit, de sorte que, du fait de la très faible inertie du capteur, la température de la couche sensible 20 décroît rapidement vers son niveau d'équilibre inférieur. Puis, lors de la phase II, qui dure respectivement 5 et 3 secondes, le chauffage est au maximum et fait monter rapidement la température de la couche 20 à son niveau d'équilibre supérieur. Une nouvelle phase I du cycle débute alors.

Plus précisément, les trois courbes de la figure 4 correspondent à une atmosphère d'air ambiant contenant respectivement 70, 150 et 400 ppm de monoxyde de carbone. Ces courbes se caractérisent par une brusque augmentation de la résistance dès que le capteur n'est plus chauffé ou chauffé à température réduite, l'augmentation étant d'autant plus grande que la concentration en monoxyde de carbone est faible. La résistance diminue ensuite lentement jusqu'à ce que le capteur soit à nouveau chauffé à température élevée. Très rapidement alors, la résistance chute fortement et reste assez constante jusqu'à ce que le chauffage soit stoppé ou réduit.

Les trois courbes de la figure 5 se rapportent à une atmosphère additionnée de méthane à des concentrations de respectivement 500, 1'000 et 10'000 ppm. On observe que ces courbes diffèrent de celles de la figure 4 par le fait que la résistance est pratiquement stable durant la phase I.

Selon l'invention, une bonne détermination de la forme de la courbe de variation de la résistance de la couche sensible est possible en effectuant quatre mesures , à savoir:

- une mesure à l'instant A lorsque la température est en train de chuter du fait de l'arrêt ou de la réduction du chauffage,

- une mesure à l'instant B lorsque la température est à son niveau d'équilibre inférieur,
- une mesure à l'instant C lorsque la température est en train de monter du fait de l'augmentation du chauffage, et
- une mesure à l'instant D lorsque la température est à son niveau d'équilibre supérieur.

Plus précisément encore, l'instant B est celui auquel le chauffage est arrêté ou réduit, tandis que l'instant D est celui auquel le chauffage est à son maximum.

Typiquement, à titre d'exemple non limitatif, l'instant A intervient 0,1 s après l'arrêt ou la réduction du chauffage, alors que l'instant C intervient 0,087 s après l'enclenchement du chauffage maximum.

Ainsi, dans le cas du monoxyde de carbone (figure 4), les quatre instants de mesure ont les valeurs suivantes:

A : 0,13 s
B : 10 s
C : 10,087 s
D :15 s

Dans le cas du méthane (figure 5), les quatre instants de mesure ont les valeurs suivantes:

A : 0,13 s
B :7 s
C : 7,087 s
D : 10 s

Les moyens de mesure 44 envoient donc, à chaque cycle, aux moyens de traitement 60 les mesures de résistance effectuées aux quatre instants A, B, C et D. Comme déjà mentionné, ces moyens de traitement élaborent, à partir des quatre mesures, des informations représentatives, pour chaque cycle, de la forme de la courbe de variation de la résistance de la couche sensible.

Plus précisément, ces informations représentatives sont les suivantes:

$$- X = R_B - R_A$$

$$- Y = R_D - R_C$$

$R_A$, $R_B$, $R_C$ et $R_D$ étant les valeurs de la résistance mesurée respectivement aux instants A, B, C et D.

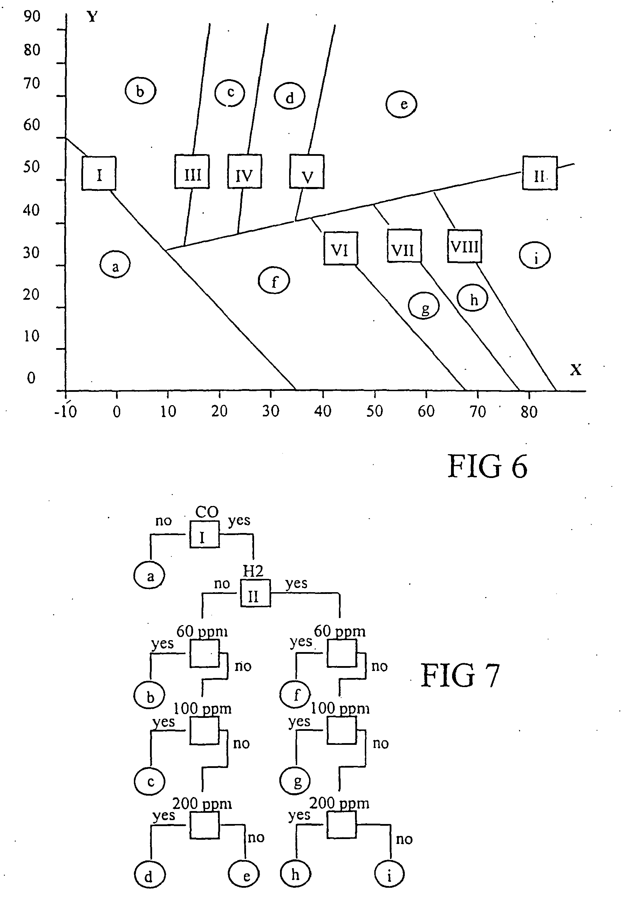

La figure 6 va servir à expliquer la manière dont les moyens d'analyse 64, avec l'aide de la mémoire 62, exploitent ces informations pour déterminer la concentration du gaz recherché. Cette figure est un diagramme en coordonnées cartésiennes avec, en abscisse, la valeur de X et, en ordonnée, la valeur de Y, obtenues avec des mélanges gazeux contenant de l'hydrogène et du monoxyde de carbone à des concentrations variant respectivement de 0 à plus de 200 ppm et de 0 à plus 400 ppm, et une humidité relative comprise entre 20 et 75% à température ambiante.

Le diagramme est découpé au moyen de huit droites I à VIII se coupant et délimitant neuf domaines identifiés par les lettres a à i. Le domaine a correspond à un mélange gazeux comportant moins de 60 ppm de CO. Les domaines b à e se rapportent à des mélanges ayant moins de 25 ppm de $H_2$ et respectivement plus de 60, 100, 200 et 400 ppm de CO. Enfin, les domaines f à i caractérisent des mélanges ayant plus de 25 ppm de $H_2$ et respectivement plus de 60, 100, 200 et 400 ppm de CO.

Les informations de référence contenues dans la mémoire 62 sont un ensemble de huit couples de valeurs m et n représentant respectivement l'ordonnée à l'origine et la pente des huit droites I à VIII.

Pour un couple X et Y de valeurs mesurées, les moyens d'analyse 64 vont obtenir la valeur de la concentration du gaz correspondante en identifiant le domaine dans lequel se situe le point de coordonnées X et Y.

A cet effet, les moyens d'analyse 64 effectuent, selon l'arbre logique représenté à la figure 7, pour les huit droites, une succession de vérifications de l'équation d'inégalité:

$$Y > m + nX.$$

Plus précisément, si l'équation est vérifiée, cela signifie que le point X - Y se trouve au-dessus de la droite caractérisée par les valeurs m et n. Si, au contraire, l'équation n'est pas vérifiée, cela signifie que le point X - Y se trouve au-dessous de cette droite.

Dans une première étape, les moyens 64 déterminent si le point X - Y se trouve au-dessus ou en-dessous de la droite I. S'il est en-dessous, cela signifie que le point est dans le domaine a et que le gaz analysé contient donc moins de 60 ppm de CO. Pour ce domaine, la concentration en $H_2$ n'est pas recherchée.

Si, au contraire, le point X-Y se trouve au-dessus de la droite I, cela signifie que la concentration en CO est supérieure à 60 ppm. Dans ce cas, l'opération suivante a pour but de déterminer si le point X - Y est au-dessus ou en-dessous de la droite II.

S'il est au-dessus, c'est-à-dire dans l'un des domaines b, c, d et e, cela signifie que la concentration en $H_2$ est inférieure à 25 ppm. Dans ce cas, la suite de l'analyse se déroule selon la branche droite de l'arbre de la figure 7.

Trois opérations sont alors prévues afin de déterminer, par la vérification de l'équation d'inégalité pour les droites III, IV et V, si le point se trouve respectivement:

- dans le domaine b ou l'un des domaines c, d et e,
- dans le domaine c ou l'un des domaines d et e,
- dans le domaine d ou le domaine e.

Si le point X - Y est en-dessous de la droite II, c'est-à-dire dans l'un des domaines f, g, h et i, cela signifie que la concentration en $H_2$ est supérieure à 25 ppm. Dans ce cas, la suite de l'analyse se déroule selon la branche gauche de l'arbre de la figure 7.

Trois opérations sont alors prévues afin de déterminer, par la vérification de l'équation d'inégalité pour les droites VI, VII et VIII, si le point se trouve respectivement:

- dans le domaine f ou l'un des domaines g, h et i,
- dans le domaine g ou l'un des domaines h et i,
- dans le domaine h ou le domaine i.

On voit ainsi que, par un choix judicieux des points caractéristiques de la courbe de variation de la résistance de la couche sensible du capteur et une méthode d'analyse de type neuronique, le détecteur selon l'invention permet, avec des moyens très simples et un capteur unique, de déterminer avec précision la concentration de plusieurs gaz contenus dans un mélange.

**Revendications**

1. Détecteur de gaz, comportant:

   - un capteur (40) comprenant une couche sensible d'oxyde métallique destinée à être mise en contact avec un mélange gazeux contenant ledit gaz,
   - des moyens de chauffage (42) pour faire varier cycliquement la température de ladite couche entre un niveau d'équilibre inférieur et un niveau d'équilibre supérieur,
   - des moyens de mesure (44) pour obtenir plusieurs valeurs de la résistance de la couche pendant le cycle de chauffage, et
   - une électronique (46) de détermination de la concentration dudit gaz dans le mélange à partir desdites valeurs, ladite électronique comportant des moyens de traitement (60), pour fournir des informations représentatives de la forme de la courbe de variation de ladite résistance au cours d'un cycle, une mémoire (62), destinée à contenir des données de référence représentatives de la forme de la courbe de variation de ladite résistance pour différentes concentrations dudit gaz, ainsi que des moyens d'analyse (64) agencés pour comparer les informations fournies par lesdits moyens de traitement auxdites données de référence pour en déduire la concentration du gaz dans le mélange,

**caractérisé en ce que** lesdits moyens de mesure (44) sont agencés de manière à recueillir, lors de chaque cycle, uniquement:

- des valeurs de la résistance de la couche sensible à deux températures intermédiaires aux températures d'équilibre, à des instants prédéterminés, une fois lorsque la température est croissante et une fois lorsqu'elle est décroissante, et
- des valeurs de la résistance de la couche sensible aux deux températures d'équilibre, et

**en ce que** lesdites informations représentatives de la forme de la courbe de variation de ladite résistance au cours d'un cycle comprennent, d'une part, la différence X entre les résistances mesurées à la température d'équilibre supérieure et à ladite température croissante et, d'autre part, la différence Y entre les résistances mesurées à la température d'équilibre inférieure et à ladite température décroissante.

**2.** Détecteur selon la revendication 1, **caractérisé en ce que** ladite mémoire (62) contient des paramètres représentatifs d'une pluralité de droites découpant en plusieurs domaines un diagramme en coordonnées cartésiennes avec, en abscisse, la valeur de X et, en ordonnée, la valeur de Y, obtenues avec des mélanges gazeux contenant ledit gaz à plusieurs concentrations connues.

**3.** Détecteur selon la revendication 2, **caractérisé en ce que** lesdits paramètres sont l'ordonnée à l'origine et la pente de chacune desdites droites.

**4.** Détecteur selon l'une des revendications 2 et 3, **caractérisé en ce que** lesdits moyens d'analyse (64) sont agencés de manière à identifier le domaine dans lequel se situe le point de coordonnées X et Y correspondant aux résistances mesurées.

**5.** Détecteur selon l'une des revendications 1 à 4, **caractérisé en ce que** ledit capteur (40) est formé d'un empilement de couches comprenant au moins un substrat (30), un corps de chauffe (24) occupant une partie centrale du substrat, et une couche sensible (20) d'oxyde métallique disposée au moins partiellement au-dessus du corps de chauffe, et **en ce que** ledit substrat (30) a la forme d'un cadre grâce auquel l'épaisseur du capteur est plus faible dans sa partie centrale qu'à sa périphérie.

**Claims**

**1.** Gas detector, comprising:

- a sensor (40) comprising a sensitive metal oxide layer intended to be brought into contact with a gas mixture containing said gas;
- heating means (42) for cyclically varying the temperature of said layer between a lower equilibrium level and an upper equilibrium level;
- measurement means (44) for obtaining several values of the resistance of the layer during the heating cycle; and
- an electronic unit (46) for determining the concentration of said gas in the mixture on the basis of said values, said electronic unit including processing means (60) for delivering information representative of the shape of the curve of variation of said resistance during a cycle, a memory (62), intended to contain reference data representative of the shape of the curve of variation of said resistance for various concentrations of said gas, and analysis means (64) designed to compare the information delivered by said processing means with said reference data in order to deduce therefrom the concentration of the gas in the mixture,

**characterized in that** said measurement means (44) are designed so as to collect, during each cycle, only:

- values of the resistance of the sensitive layer at two temperatures intermediate between the equilibrium temperatures at predetermined instants, once when the temperature is increasing and once when it is decreasing; and
- values of the resistance of the sensitive layer at the two equilibrium temperatures, and

**in that** said information representative of the shape of the curve of variation of said resistance during a cycle comprise, on the one hand, the difference X between the resistances measured at the upper equilibrium temperature and at said increasing temperature and, on the other hand, the difference Y between the resistances measured at the lower equilibrium temperature and at said decreasing temperature.

**2.** Detector according to Claim 1, **characterized in that** said memory (62) contains parameters representative of a plurality of straight lines cutting, in several regions, a plot in Cartesian coordinates, with the X value plotted on the x-axis and the value of Y plotted on the y-axis, which are obtained with gas mixtures containing said gas at several known concentrations.

**3.** Detector according to Claim 2, **characterized in that** said parameters are the ordinate at the origin and the slope of each of said straight lines.

**4.** Detector according to either of Claims 2 and 3, **characterized in that** said analysis means (64) are designed so as to identify the region in which the point of coordinates X and Y, corresponding to the measured resistances, lies.

**5.** Detector according to one of Claims 1 to 4, **characterized in that** said sensor (40) is formed from a multilayer stack comprising at least one substrate (30), a heater (24) occupying a central portion of the substrate, and a metal oxide sensitive layer (20) deposited at least partly on top of the heater, and **in that** said substrate (30) has the form of a frame whereby the thickness of the sensor is smaller in its central portion than on its periphery.

**Patentansprüche**

**1.** Gasdetektor, umfassend:

- einen Sensor (40), der eine empfindliche Metalloxidschicht aufweist, die dazu bestimmt ist, mit einem Gasgemisch, das das Gas enthält, in Kontakt gebracht zu werden,
- Mittel zum Heizen (42), um die Temperatur der Schicht zyklisch zwischen einem unteren und einem oberen Gleichgewichtsniveau variieren zu lassen,
- Messmittel (44), um mehrere Werte des Widerstands der Schicht während des Heizzyklus zu erhalten,
- eine Elektronik (46) zum Bestimmen der Konzentration des Gases in dem Gemisch ausgehend von den Werten, wobei die Elektronik Verarbeitungsmittel (60) aufweist, um Informationen zu liefern, die für die Form der Variationskurve des Widerstands im Laufe eines Zyklus repräsentativ sind, einen Speicher (62), der dazu bestimmt ist, Referenzdaten zu enthalten, die für die Form der Variationskurve des Widerstands für verschiedene Konzentrationen des Gases repräsentativ sind, sowie Analysemittel (64), die eingerichtet sind, um die von den Verarbeitungsmitteln gelieferten Informationen mit den Referenzdaten zu vergleichen, um daraus die Konzentration des Gases in dem Gemisch abzuleiten,

**dadurch gekennzeichnet, dass** die Messmittel (44) so eingerichtet sind, dass sie bei jedem Zyklus nur

- Werte des Widerstands der empfindlichen Schicht bei zwei Zwischentemperaturen zwischen den Gleichgewichtstemperaturen in vorbestimmten Augenblicken sammeln, einmal, wenn die Temperatur steigt, und einmal, wenn sie sinkt, und
- Werte des Widerstands der empfindlichen Schicht bei den zwei Gleichgewichtstemperaturen sammeln, und

**dadurch**, dass die für die Form der Variationskurve des Widerstands im Laufe eines Zyklus repräsentativen Informationen einerseits den Unterschied X zwischen den bei der oberen Gleichgewichtstemperatur und der steigenden Temperatur gemessenen Widerständen enthalten und andererseits den Unterschied Y zwischen den bei der unteren Gleichgewichtstemperatur und der sinkenden Temperatur gemessenen Widerständen enthalten.

**2.** Detektor nach Anspruch 1, **dadurch gekennzeichnet, dass** der Speicher (62) Parameter enthält, die für mehrere Geraden repräsentativ sind, die ein kartesisches Koordinatendiagramm mit auf der Abszisse dem Wert von X und auf der Ordinate dem Wert von Y, die mit Gasgemischen erzielt werden, die das Gas mit mehreren bekannten Konzentrationen enthalten, in mehrere Bereiche einteilen.

**3.** Detektor nach Anspruch 2, **dadurch gekennzeichnet, dass** die Parameter der Y-Achsenabschnitt und das Gefälle jeder der Geraden sind.

**4.** Detektor gemäß einem der Ansprüche 2 und 3, **dadurch gekennzeichnet, dass** die Analysemittel (64) so eingerichtet sind, dass sie den Bereich identifizieren, in dem sich der X- und Y-Koordinatenpunkt befindet, der den gemessenen Widerständen entspricht.

**5.** Detektor nach einem der Ansprüche 1 bis 4, **dadurch gekennzeichnet, dass** der Sensor (40) aus einer Stapelung von Schichten ausgebildet ist, die mindestens ein Substrat (30), einen Heizkörper (24), der einen zentralen Teil des Substrats belegt, und eine empfindliche Schicht (20) aus Metalloxid, die zumindest teilweise über dem Heizkörper liegt, aufweist, und dass das Substrat (30) die Form eines Rahmens hat, dank dem die Stärke des Sensors in seinem zentralen Teil geringer ist als an seiner Peripherie.

FIG 1

22a 22 20 36 34 28 14

18 28a

16

12

30 24 38 32 26

10

FIG 2

FIG 3

FIG 4

FIG 5

FIG 6

FIG 7